# EUROPEAN PATENT SPECIFICATION

(11) **EP 4 079 718 B1**
(45) Date of publication and mention of the grant of the patent: **15.07.2026**
(21) Application number: 22167440.1
(22) Date of filing: 08.04.2022
(51) Int. Cl.: C07C 29/151, C07C 31/04, C01B 3/00, C10J 3/00

(54) **PROCESS FOR THE PREPARATION OF BIOMETHANOL**
VERFAHREN ZUR HERSTELLUNG VON BIO-METHANOL
PROCÉDÉ POUR LA PRÉPARATION DE BIOMÉTHANOL

(30) Priority: 12.04.2021 FI 20210020
(43) Date of publication of application: 26.10.2022
(73) Proprietor: Janhunen, Timo, 00330 Helsinki (FI)
(72) Inventor: Janhunen, Timo, 00330 Helsinki (FI)
(74) Representative: Laine IP Oy

(56) References cited:
- US-A1- 2013 345 325
- MARIO TOLEDO T. ET AL: "Syngas production from coal in presence of steam using filtration combustion", INTERNATIONAL JOURNAL OF HYDROGEN ENERGY, vol. 40, no. 19, 1 May 2015 (2015-05-01), AMSTERDAM, NL, pages 6340 - 6345, XP055959818, ISSN: 0360-3199, DOI: 10.1016/j.ijhydene.2015.03.022
- DAVID S NEWSOME: "The water-gas shift reaction", CATALYSIS REVIEWS: SCIENCE AND ENGINEERING, MARCEL DEKKER INC. NEW YORK, US, vol. 21, no. 2, 16 September 1980 (1980-09-16), pages 275 - 318, XP008176054, ISSN: 0161-4940, [retrieved on 20061205], DOI: 10.1080/03602458008067535

## Description

### Field of invention

The present invention relates to a method according to the preamble of claim 1 for producing climate-friendly engine fuel.

### Background

Global warming is the most significant problem the humankind is facing in 2021, and it is mainly related to the increase of CO2-concentration in the atmosphere caused by combustion of fossil fuels. The aim in, for example, road traffic is to reduce CO2 emissions by increasing the amount of electric cars, using hydrogen as fuel as well as synthetic, so-called e-fuels, the carbon of which is extracted from the atmosphere. Both of these methods, however, require large amounts of electric energy, whereby they are expensive methods.

Currently there are about 1.3 billion cars with internal combustion engine in the world, and most of them are relatively easy to convert to run on climate-friendly biofuels, such as biomethanol. An internal combustion engine does not cause global warming as such. Instead the culprit is the fossil carbon contained by the fuel of the engine that forms during combustion CO2, which is a powerful greenhouse gas.

One example of producing methanol and hydrocarbons is disclosed in US 2013/0345325.

### Purpose of the invention

When the fossil carbon contained by the fuel is replaced with renewable biocarbon, the CO2 formed during combustion of fuel does not harm the atmosphere. The present invention is based on production of biocarbon-based biomethanol which can in many instances able replace fossil carbon-containing engine fuels economically, thus considerably reducing the CO2 load of the atmosphere.

### Description of the invention

The present invention of producing biocarbon-based biomethanol is based on a novel way of producing climate-friendly biomethanol based on biocarbon, such as biocarbon pellets, to be used as engine fuel. The method is based on the following chemical reactions, in which the carbon is renewable biocarbon:

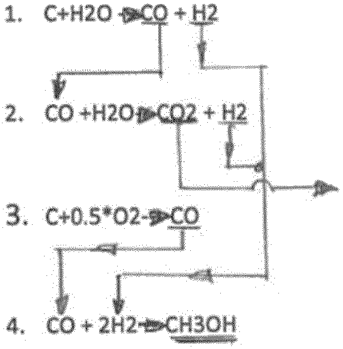

Reaction 1 is a reaction in which water vapour is introduced into glowing biocarbon ember. In reaction 2 the gas mixture is directed through catalysts in a temperature of, e.g. 400 C. In reaction 3 biocarbon is burned under oxygen-deficient condition. In reaction 4 biomethanol is formed by means of catalysts.

This reaction chain 1 to 4 based on biocarbon has never before been disclosed, it is a new and highly important method of producing climate-friendly CO2-neutral biomethanol. In these reactions, no hydrogen is lost in reduction reactions.

In the method of the above-described rection chain the production of climate-friendly biocarbon-based biomethanol the production of the fuel is based on the use of biocarbon, such as biocarbon pellets, whereby the use of the produced engine fuel is not harmful to the atmosphere, as the formed CO2 is originally from renewable biocarbon. The methods and catalysts used in reactions 2 and 4 are known in the industry, and no expensive hydrogen is lost in reduction reactions in the production of biomethanol according to the reaction chain 1 to 4, and the CO2 produced as a by-product is not harmful to the atmosphere as the carbon contained therein is renewable biocarbon.

When producing biocarbon-based biomethanol engine fuel according to the reaction chain 1 to 4 no hydrogen is lost in reduction in the production of biomethanol. Thus, the increase of CO2 concentration in the atmosphere is reduced when using this fuel as engine fuel, because the CO2 formed during combustion of the fuel is based on renewable biocarbon.

## Claims

1. A method of producing engine fuel, the base materials being biocarbon and the reactions
occurring according to the formula 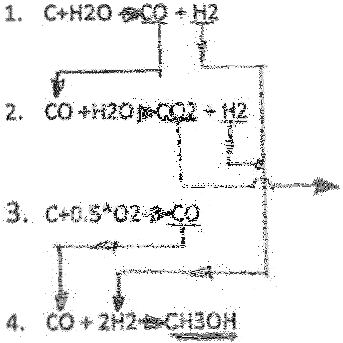 wherein:
in reaction 1 water vapour is introduced into glowing biocarbon ember,
in reaction 2 the gas mixture is directed through catalysts in a temperature of 400 C,
in reaction 3 biocarbon is combusted in oxygen-deficient conditions and
in reaction 4 biomethanol is formed by means of catalysts, **characterized in that**
- in reaction 3 biocarbon is combusted with oxygen (O2), and
- the hydrogen formed in reactions 1 and 2 is used in reaction 4.

## Patentansprüche

1. Verfahren zum Produzieren von Motorkraftstoff, wobei die Basismaterialien Biokohlenstoff sind und die Reaktionen stattfinden gemäß der Formel 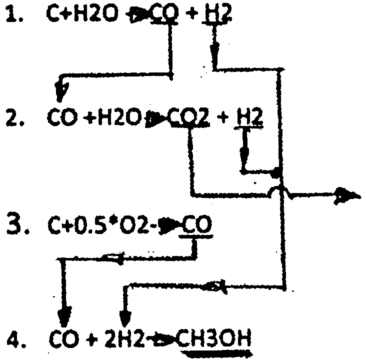 wobei:
in Reaktion 1 Wasserdampf in glühende Biokohlenstoffglut eingeleitet wird,
in Reaktion 2 das Gasgemisch bei einer Temperatur von 400 °C durch Katalysatoren geleitet wird,
in Reaktion 3 Biokohlenstoff unter sauerstoffarmen Bedingungen verbrannt wird und
in Reaktion 4 Biomethanol mittels Katalysatoren gebildet wird, die **dadurch gekennzeichnet sind, dass**
- in Reaktion 3 Biokohlenstoff mit Sauerstoff (02) verbrannt wird und
- der in den Reaktionen 1 und 2 gebildete Wasserstoff in Reaktion 4 verwendet wird.

## Revendications

1. Procédé de production de carburant pour moteurs, dont les matières premières sont du biocarbone et les réactions se déroulant selon la formule 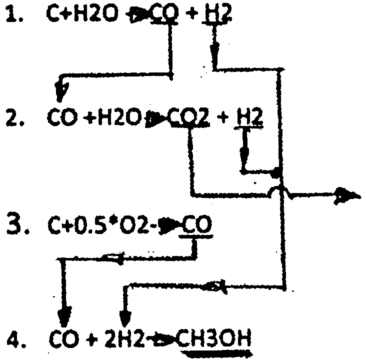 dans lequel :
dans la réaction 1, de la vapeur d'eau est introduite dans une braise de biocarbone incandescente,
dans la réaction 2, le mélange gazeux est dirigé à travers des catalyseurs à une température de 400 °C,
dans la réaction 3, le biocarbone est brûlé dans des conditions pauvres en oxygène et
dans la réaction 4, le biométhanol est formé au moyen de catalyseurs, **caractérisés en ce que**
- dans la réaction 3, le biocarbone est brûlé avec de l'oxygène (O2), et
- l'hydrogène formé dans les réactions 1 et 2 est utilisé dans la réaction 4.
